# FASCICULE DE BREVET EUROPEEN

(11) **EP 2 276 457 B1**
(45) Date de publication et mention de la délivrance du brevet: **29.11.2017**
(21) Numéro de dépôt: 09731998.2
(22) Date de dépôt: 16.04.2009
(51) Int. Cl.: A61K 8/97, A61Q 19/02

(54) **UTILISATION D'UN EXTRAIT DE MYRTE COMME DÉPIGMENTANT**
VERWENDUNG VON MYRTENEXTRAKT ALS DEPIGMENTIERUNGSMITTEL
USE OF MYRTLE EXTRACT AS DEPIGMENTING AGENT

(30) Priorité: 16.04.2008 FR 0852570
(43) Date de publication de la demande: 26.01.2011
(73) Titulaire: Pierre Fabre Dermo-Cosmétique, 92100 Boulogne-Billancourt (FR)
(72) Inventeur: FABRE, Bernard, F-31450 Belberaud (FR)
(74) Mandataire: Regimbeau
(86) Numéro de dépôt international: PCT/EP2009/054487
(87) Numéro de publication internationale: WO 2009/127673

(56) Documents cités:
- EP-A- 1 316 306
- WO-A-2007/118605
- WO-A-2008/007004
- WO-A-2008/015127
- FR-A- 2 735 026
- FR-A- 2 783 425
- FR-A- 2 787 710
- FR-A- 2 810 544
- FR-A- 2 890 860
- JP-A- 2006 199 678
- US-A1- 2006 216 254
- US-A1- 2007 025 939
- US-B1- 6 365 137
- DATABASE WPI Week 200647 Thomson Scientific, London, GB; AN 2006-458551 XP002506735 & JP 2006 169133 A (MARUZEN SEIYAKU KK) 29 juin 2006 (2006-06-29)
- DATABASE GNPD [en ligne] MINTEL Mars 2006 'Brightening Moisture Lotion' Database accession no. 432364
- DATABASE GNPD [en ligne] MINTEL Février 2007 'Complete Regulating Care' Database accession no. 654927
- DATABASE GNPD [en ligne] MINTEL Décembre 2001 'Look Alive Moisture Cream' Database accession no. 124861

## Description

La présente invention concerne une utilisation cosmétique d'un extrait de myrte comme agent dépigmentant dans une composition cosmétique

La description porte sur un procédé cosmétique de blanchiment et/ou d'éclaircissement de la peau et/ou des poils et/ou des cheveux, ainsi qu'un procédé cosmétique pour réduire et/ou supprimer et/ou prévenir les taches de pigmentation de la peau, comprenant l'application sur la peau et/ou les poils et/ou les cheveux d'une composition cosmétique selon l'invention.

### D'une part :

La pigmentation de la peau est due à la faculté de certaines cellules spécialisées, les mélanocytes, à synthétiser la mélanine. Cette dernière est un colorant qui peut prendre différentes formes moléculaires, la mélanine et ses dérivés, pigments brun noir, la phaeomélanine et ses dérivés, pigments de couleur rouge-jaune. Les mélanocytes sont localisés dans les couches basales de l'épiderme, ils possèdent des prolongements cytoplasmiques ou dendrites nécessaires au transport de la mélanine. Cette dernière se présente sous forme de grains contenus dans les mélanosomes. Elle y est synthétisée à partir de tyrosine par l'action en particulier d'une enzyme, la tyrosinase. Les mélanosomes formés migrent dans les kératinocytes grâce aux prolongements dendritiques, puis pénètrent dans ces cellules par un phénomène de translocation. A mesure que les kératinocytes se différencient en cornéocytes, la mélanine se disperse dans le cytoplasme et est éliminée par desquamation.

L'inhibition de la tyrosinase est une cible importante pour les produits qui visent une activité dépigmentante. Par cette inhibition, on bloque la mélanogénèse. Ceci est le cas pour de nombreux principes actifs comme l'arbutine ou l'hydroquinone. Cette activité peut être observée *in vitro* directement sur l'enzyme ou d'une façon plus globale sur des mélanocytes dont on évaluera la faculté à synthétiser la mélanine. La faculté à empêcher le transport des mélanosomes est également une cible intéressante pour un produit dépigmentant. Ceci pourra être observé par la faculté ou non d'un produit à empêcher la formation des dendrites indispensables à la migration de ces mélanosomes et donc de la mélanine qui les compose.

### D'autre part :

Le myrte, ou *Myrtus communis,* appartient à la famille des Myrtacées. C'est un arbuste qui peut atteindre 2 à 3 mètres de haut, ses tiges sont irrégulières et sont recouvertes d'une écorce rousse presque lisse.

Les feuilles sont opposées, persistances, coriaces, vert foncé. Le limbe est ovale, aigu au sommet, parsemé de ponctuations glanduleuses translucides, il s'atténue en un très court pétiole.

Les fleurs sont blanches, odorantes et solitaires, portées sur un long pédoncule à l'aisselle des feuilles. Elles s'épanouissent de mai à juillet. Le calice est soudé à l'ovaire dans sa partie inférieure. Il présente 5 divisions triangulaires, étalées, plus courtes que les pétales. La corolle a 5 pétales libres entre eux. Les étamines sont libres et nombreuses. L'ovaire est trioculaire et surmonté d'un style terminé par un stigmate unique. Les 3 loges de l'ovaire renferment chacune de nombreux ovules. Le fruit est une baie charnue, ovoïde, noir bleuâtre à maturité. Il est surmonté des 5 dents persistantes du calice. Il renferme un grand nombre de graines dépourvues d'albumen.

Le myrte croît indifféremment en terrains calcaires ou siliceux. Il craint le gel et ne s'élève donc pas à une grande altitude. Il est très commun en France sur le littoral méditerranéen, surtout en Provence. Il habite également l'Europe méridionale, les régions occidentales et méridionales de l'Asie, le nord de l'Afrique.

Le myrte est surtout utilisé pour sa richesse en huile essentielle présente dans les feuilles, mais aussi dans le fruit. Cette essence, qui s'extrait par distillation à la vapeur ou par extraction avec des solvants apolaires comme l'hexane, est de couleur verdâtre et d'odeur agréable. Elle est composée d'eucalyptol, de linalol, géraniol, pinène et limonène et de substances plus spécifiques comme le myrtol et le myrténol.

Les feuilles renferment également de fortes proportions de tanins, galliques en majorité, 14 % en moyenne, ainsi que des flavonoïdes (myricétol, kaempferol et leurs dérivés glycosilés), des coumarines (aesculine, aesculetine) et des acides phénoliques.

Les fruits, en plus des huiles essentielles, contiennent une grande quantité de composés polyphénoliques dont une partie est constituée de tanins hydrolysables et condensés. On trouve également des composés phénoliques plus simples comme la quercétine, la patulétine, les acides galliques et ellagiques.

Traditionnellement, le myrte est utilisé pour son huile essentielle. Ainsi G. GARNIER et ses co-auteurs décrivent dans leur ouvrage : Ressources Médicinales de la Flore Française, édité par Vigot Frères à Paris en 1961, que «L'huile essentielle, administrée autrefois sous le nom impropre de myrtol, douée de propriétés antiseptiques et désinfectantes, agit comme stimulant de la digestion, est hémostatique et en plus exerce une action marquée sur les affections cutanées squameuses et principalement sur le psoriasis ». L'indication sur le psoriasis est également décrite dans le brevet FR 2 783 425 intitulé « Extrait de myrte titré en myrtucommulone B', son procédé de préparation et son application en dermatologie et en cosmétologie ».

L'utilisation en tant que déodorant d'une composition à base d'huile essentielle du myrte, en association avec d'autres plantes, a également fait l'objet de la demande de brevet US2001006626.

La demande JP2001220312 décrit une eau distillée hydratante comprenant de l'huile essentielle de myrte.

Toujours en cosmétologie mais dans un usage capillaire, les extraits de myrte, seuls ou associés, ont fait l'objet de travaux comme antipelliculaire : « Composition capillaire comprenant un extrait de myrte, son procédé de préparation et son utilisation notamment pour un traitement antipelliculaire : FR 2 735 026, et « Association extrait de myrte et antifongique » : FR 2 741 265.

La présente invention propose un nouvel actif dépigmentant issu d'un extrait végétal : le myrte ou *Myrtus communis.* De façon tout à fait surprenante et innovante, la Demanderesse a mis en évidence qu'un extrait de myrte est capable d'inhiber la synthèse et le transport de la mélanine par les mélanocytes.

La présente invention concerne l'utilisation d'un extrait de myrte dans une composition cosmétique comme agent dépigmentant. La présente description porte sur un procédé cosmétique de blanchiment et/ou d'éclaircissement de la peau et/ou des poils et/ou des cheveux, ainsi qu'un procédé cosmétique pour réduire et/ou supprimer et/ou prévenir les taches de pigmentation de la peau, comprenant l'application sur la peau et/ou les poils et/ou les cheveux d'une composition cosmétique comprenant un extrait de myrte.

La présente description porte également sur l'utilisation d'un extrait de myrte pour la fabrication d'une composition dermatologique destinée à dépigmenter la peau.

Enfin dans le cadre de la présente invention les compositions cosmétiques comprennent, à titre de principe actif, un extrait de myrte dans un milieu physiologiquement acceptable, avantageusement en association avec au moins un autre agent actif tel que l'ascorbyl glucoside.

Dans le cadre de la présente invention, l'extrait de myrte peut être préparé selon des étapes classiques d'extraction et de concentration connues de l'Homme du Métier.

L'extrait de myrte peut être obtenu à partir de la plante entière ou d'une partie de plante. De manière particulièrement avantageuse selon la présente invention, l'extrait de myrte est obtenu à partir des feuilles, des fruits, ou d'un mélange des feuilles et des fruits de myrte.

Ainsi, la fabrication d'un extrait de myrte à partir de feuilles et/ou de fruits de myrte, peut comprendre les étapes suivantes :
- un broyage des feuilles et/ou des fruits de myrte,
- une extraction des feuilles et/ou des fruits de myrte broyés par un solvant, tel qu'un solvant polaire,
- une récupération de la solution d'extraction par filtration ou par essorage,
- une concentration de la solution récupérée par évaporation, pour obtenir l'extrait de myrte à mélanger, le cas échéant, à d'autres constituants, afin d'obtenir ladite composition.

De préférence, les feuilles et/ou les fruits de myrte sont des feuilles et/ou des fruits de myrte secs.

Plus préférentiellement, le rapport en poids des feuilles aux fruits de myrte est voisin de 90/10.

Dans l'étape d'extraction, le solvant utilisé est avantageusement un solvant polaire, tel qu'un alcool en C1 à C4 ou l'acétone, ou un mélange eau-alcool ou un mélange acétone-eau.

Le rapport en poids des feuilles et/ou des fruits au solvant est typiquement compris entre 1 et 20, et de préférence entre 1 et 4, et la température d'extraction est généralement comprise entre la température ambiante et la température d'ébullition du solvant. La durée d'extraction est typiquement comprise entre 1 heure et 24 heures.

Selon un mode avantageux de mise en oeuvre du procédé selon l'invention, la concentration de la solution récupérée par évaporation est réalisée à pression réduite, à une température comprise entre 40 et 100°C jusqu'à l'obtention d'une poudre. On peut également, lors de l'opération de concentration, ajouter un solvant à haut point d'ébullition et plus particulièrement la glycérine, le propylène glycol, le butylène glycol ou le transcutol. L'extrait de myrte est alors sous forme liquide.

Avantageusement, l'extrait de myrte comprend des polyphénols en une quantité comprise entre 1 et 50 %, de préférence entre 10 et 40 %, et plus préférentiellement encore entre 20 et 30 % en poids sec, par rapport à la matière sèche de l'extrait.

Parmi les polyphénols, on trouve typiquement des flavonoïdes, ainsi que des tannins.

Selon un mode de réalisation avantageux selon l'invention, l'extrait de myrte comprend des flavonoïdes en une quantité comprise entre 0,1 et 5 %, de préférence entre 1 et 4 %, et plus préférentiellement encore entre 2 et 3 % en poids sec, par rapport à la matière sèche de l'extrait.

Selon un autre mode de réalisation avantageux selon l'invention, l'extrait de myrte comprend des tanins en une quantité comprise entre 1 et 50 %, de préférence entre 5 et 40 %, et plus préférentiellement encore entre 10 et 30 % en poids sec, par rapport à la matière sèche de l'extrait.

Selon une caractéristique particulière de la présente invention, l'extrait de myrte comprend des flavonoïdes en une quantité comprise entre 0,5 et 4 %, typiquement entre 1 et 3,5 %, ainsi que des tannins en une quantité comprise entre 5 et 40 %, typiquement entre 10 et 40 %, par exemple entre 20 et 40 %.

L'objet de la présente invention concerne l'utilisation d'un extrait de myrte comme agent dépigmentant dans une composition cosmétique.

Avantageusement, la composition cosmétique dans le cadre de la présente 'invention est destinée à blanchir et/ou éclaircir la peau et/ou les poils et/ou les cheveux.

L'utilisation d'un extrait de myrte selon la présente invention permet ainsi d'unifier le teint : ce qui se caractérise par un teint de peau uniforme, plus clair, plus transparent, plus lumineux. L'éclat du teint se trouve donc amélioré.

Les avantages obtenus avec la composition dans le cadre de la présente invention sont particulièrement intéressants pour les peaux sensibles, quelle que soit leur nature (sèche, normale, grasse), et plus particulièrement pour les peaux sensibles ternes et sans éclat.

L'extrait de myrte, en tant qu'agent dépigmentant, a également montré de bonnes capacités :
- à atténuer visiblement l'intensité et la taille des taches pigmentaires;
- à réguler et/ou inhiber la production des mélanines, responsables de la pigmentation ;
- à estomper les taches visibles ;
- à éviter l'apparition de taches supplémentaires ;
démontrant son intérêt dans la dépigmentation de certaines taches pigmentaires inesthétiques dues à une hyperpigmentation épidermique: telles que les taches de sénescence de la peau notamment.

La présente description concerne l'utilisation d'un extrait de myrte pour la fabrication d'une composition, telle qu'une composition cosmétique ou dermatologique, destinée à dépigmenter la peau et/ou les poils et/ou les cheveux.

L'utilisation d'un extrait de myrte selon la présente invention permet avantageusement :
- de réduire et/ou supprimer les taches de pigmentation, telles que les taches d'hyperpigmentation dues à un stress pro-inflammatoire, par exemple les taches pigmentaires brunâtres induites par des UV, ou encore réduire et/ou supprimer les chloasmas ;
- de réduire et/ou inhiber la production des mélanines, responsables de la pigmentation.

L'extrait de myrte selon la présente invention peut ainsi être utilisé avantageusement dans une composition, telle qu'une composition cosmétique, pour réduire et/ou supprimer les taches de sénescence de la peau, ou encore pour réduire et/ou supprimer les taches pigmentaires brunâtres pouvant être induites par les UV, ou les chloasmas.

Dans le cadre de la présente invention les compositions cosmétiques comprennent, à titre de principe actif, un extrait de myrte dans un milieu physiologiquement acceptable ; c'est à dire compatible avec la peau et/ou le cuir chevelu, les muqueuses, les cheveux, les poils et/ou les yeux.

Préférentiellement, dans le cadre de la présente invention la composition cosmétique comprend une quantité d'extrait de myrte, à titre de principe actif, comprise entre 1 mg et 50 g, et plus préférentiellement entre 10 mg et 10 g pour 100 g de ladite composition.

Avantageusement, ladite quantité d'extrait de myrte est comprise entre 50 mg et 1 g pour 100 g de composition. De façon encore plus avantageuse, ladite quantité d'extrait de myrte est comprise entre 100 mg et 500 mg pour 100 g de composition.

Et de façon préférée, lesdites compositions comprennent en outre au moins un deuxième agent actif dépigmentant.

Ledit ou lesdits autres agents qui peuvent être ajoutés à la présente composition sont connus de l'Homme du Métier, qui pourra ajuster les proportions relatives de chaque constituant de la composition afin d'optimiser l'efficacité de ladite composition.

Avantageusement, on citera à titre indicatif et non limitatif des agents actifs choisis dans le groupe formé par la vitamine C et ses dérivés dont l'ascorbyl glucoside, l'acide salicylique, l'acide lactique, l'acide glycolique, l'acide malique, l'arbutine, l'acide kojique, la vitamine B3, l'acide linoléique et les huiles végétales en contenant, le rétinaldéhyde et ses dérivés, le résorcinol ainsi que des extraits de plante, en particulier de busserole, de réglisse, de mûrier, d'arbousier, de broussonetia, de camomille ou de scutellaire.

De préférence, la composition comprend un extrait de myrte (*Myrtus* communis) et également, dans un milieu physiologiquement acceptable, au moins un autre agent dépigmentant choisi dans le groupe constitué par l'ascorbyl glucoside, l'acide lactique, l'acide glycolique, l'acide malique, l'arbutine, l'acide kojique, l'acide linoléique et les huiles végétales en contenant, le rétinaldéhyde et ses dérivés, le résorcinol, un extrait de busserole, un extrait de réglisse, un extrait de mûrier, un extrait d'arbousier, un extrait de broussonetia, un extrait de camomille et un extrait de scutellaire, utilisé seul ou en mélange.

De manière particulièrement préférée, la composition comprend un extrait de myrte (*Myrtus* communis) et également, dans un milieu physiologiquement acceptable, au moins un autre agent dépigmentant choisi dans le groupe constitué par l'ascorbyl glucoside, l'acide glycolique, l'acide malique, l'arbutine, l'acide kojique, l'acide linoléique et les huiles végétales en contenant, le rétinaldéhyde et ses dérivés, un extrait de busserole, un extrait de réglisse, un extrait de mûrier, un extrait d'arbousier, un extrait de broussonetia et un extrait de scutellaire, utilisé seul ou en mélange.

De manière particulièrement avantageuse, la composition comprend un extrait de myrte en association avec l'ascorbyl glucoside.

Les compositions qui comprennent un extrait de myrte en association avec au moins un autre agent actif dépigmentant sont utilisées avantageusement dans toutes les applications mentionnées ci-dessus.

La composition cosmétique peut avantageusement se présenter sous toutes les formes galéniques normalement utilisées dans les domaines cosmétique et dermatologique pour une application topique ou orale.

Préférentiellement, la forme topique peut être notamment sous forme :
- d'une solution aqueuse, hydroalcoolique, éventuellement gélifiée,
- d'une dispersion du type lotion éventuellement biphasée,
- d'une émulsion huile-dans-eau ou eau-dans-huile ou multiple,
- d'un gel aqueux,
et peut avoir l'aspect d'un sérum, d'une crème, d'un gel, d'une pommade, d'un lait, d'une lotion, d'une pâte ou d'une mousse. Elle peut aussi être appliquée sous forme d'aérosol ou encore sous forme solide, et par exemple sous forme de stick.

Un des avantages de la présente invention réside dans le fait que les compositions du cadre de l'invention présentent une bonne tolérance cutanée, même pour les peaux sensibles, quelle que soit leur nature (sèche, normale, grasse).

Cette composition peut aussi se présenter sous une forme orale, telle qu'un comprimé, gélule, poudre pour suspensions buvables.

La composition peut également comprendre tous les constituants usuellement employés dans l'application envisagée. On peut notamment citer l'eau, les solvants, les huiles d'origine minérale, animale et/ou végétale, les cires, les pigments, les filtres chimiques ou minéraux, les antioxydants, les charges, les tensioactifs, les stabilisants, les conservateurs, les parfums, et les matières colorantes.

Le choix et/ou la quantité du ou des ingrédients seront également déterminés par les besoins spécifiques de la peau et/ou des poils et/ou des cheveux sur lesquels la composition sera appliquée, ainsi que par les propriétés et la consistance souhaitée pour la composition selon la présente invention.

L'invention pourra être mieux comprise à l'aide des exemples non limitatifs qui suivent et qui constituent des modes de réalisation particuliers des compositions cosmétiques et/ou dermatologiques selon l'invention.

### Exemples 1-4: préparation d'un extrait de myrte

### Exemple 1

100 kg de feuilles et fruits secs de myrte sont broyés puis extraits par 700 kg d'éthanol à 50 %. L'extraction est réalisée sous reflux pendant 1 heure et sous agitation. Les solutions sont récupérées après filtration, puis concentrées sous pression réduite à 60°C. La concentration puis le séchage sont menés jusqu'à l'obtention d'une matière sèche dont la teneur en polyphénols se situe entre 20 et 40 % et celle des flavonoïdes entre 1,5 et 3,5 %.

### Exemple 2

Les premières étapes sont identiques à celles de l'exemple 1, mais on procède différemment pour les dernières étapes de concentration et de séchage. Après extraction, lors de la concentration, on rajoute 200 kg de propylène glycol, et la concentration est menée jusqu'à l'obtention d'une solution pesant 220 kg. La teneur en matière sèche se situe entre 8 et 12 %, celle des polyphénols par rapport à la matière sèche entre 20 et 40 %, celle des flavonoïdes par rapport à la matière sèche, entre 1,5 et 3,5 %.

### Exemple 3

On procède ici à partir de matière première différente de celle de l'exemple 1. On procède aussi au moyen d'un autre solvant d'extraction de manière à obtenir un extrait présentant des teneurs différentes en flavonoïdes et en polyphénols.

1 kg de feuilles sèches, broyées, sont extraites par 10 kg de méthanol à froid pendant 12 heures. La solution méthanolique est concentrée sous vide à 60°C, puis séchée. L'extrait sec obtenu est broyé. La poudre titre entre 30 à 40 % en polyphénols et entre 3 et 4 % de flavonoïdes.

### Exemple 4

10 kg de feuilles et fruits broyés sont extraits par un mélange d'eau et d'éthanol dans des proportions de 80 à 20 sous reflux et sous agitation pendant 3 heures. La solution obtenue est essorée puis filtrée.

Sa teneur en polyphénols par rapport à la matière sèche se situe entre 5 et 10 %. Les flavonoïdes varient de 0,5 à 1,5 %.

### Exemple 5 : Evaluation de l'activité dépigmentante :

### 5.1 Activité sur la dendricité des mélanocytes en culture

La pigmentation de la peau résulte d'interactions entre les mélanocytes épidermiques et les kératinocytes. Les dendrites des mélanocytes jouent un rôle important dans le processus de pigmentation. Ils permettent le transfert des mélanosomes dans les parties basales et suprabasales de l'épiderme. Ils sont composés d'une association de microtubules et de filaments d'actine qui permettent le déplacement des grains de mélanine de l'intérieur des mélanocytes vers l'extrémité des dendrites et sa distribution au niveau de la peau (Hara et al., J Invest Dermatol, 2000).

La différenciation des mélanocytes se traduit par une stimulation de la mélanogénèse et une dendricité. Ce processus est induit par plusieurs facteurs induisant l'AMPc : α-MSH, Forskoline (Busca et al, 2000).

L'appréciation morphologique directe de l'effet d'agents dépigmentants ou stimulant la pigmentation sur la modification de la dendricité mélanocytaire se fait par marquage des filaments d'actine des mélanocytes à la phalloidine-rhodamine.

Dans cette étude, la Demanderesse a évalué l'effet de l'extrait myrte sur la modification de la dendricité des mélanocytes prétraités ou non à l'α-MSH. L'agent dépigmentant de référence utilisé est le trio de Kligmann.

Ce dernier est composé d'un mélange de 3 actifs à l'hydroquinone (1 %), l'hydrocortisone (0,5 %) et l'acide rétinoïque (0,0125 %). Pour le test, les suspensions de mélanocytes sont placées sur des lames de verre pour adhésion, puis traitées ou non pendant une heure à l'αMSH et exposées aux différentes concentrations d'extrait de myrte tel que défini dans l'exemple 1 pendant une heure. Les cellules sont ensuite traitées à un révélateur coloré, la phalloidine-TRITC de façon à révéler leur réseau d'actine et ainsi leur dendricité, puis elles sont observées au microscope de façon à observer leur morphologie.

Les mélanocytes traités uniquement à l'αMSH montrent une augmentation de leur dendricité mélanocytaire par rapport aux cellules non traitées montrant ainsi l'effet pro pigmentant de l'αMSH. Par contre, les mélanocytes prétraités à l'αMSH puis au trio de Kligmann dilué au 1/75000 ou prétraité à l'extrait de myrte à 0,3 µg/ml ou à 1 µg/ml ne montrent pas d'augmentation de leur dendricité ou très légèrement.
Ces 2 substances montrent ainsi un effet inhibiteur à l'αMSH facteur d'endogène de la stimulation de la biosynthèse de la mélanine, et ainsi leur capacité à montrer une activité dépigmentante.

### 5.2 Activité sur la mélanogénèse

Les mélanocytes sont mis à incuber dans un milieu de culture adéquat à 37°C en contact pendant 72 heures avec l'extrait de myrte selon l'exemple 1 à différentes concentrations ainsi qu'avec l'hydroquinone, substance de référence. A la fin de la période d'incubation, les mélanocytes sont prélevés, lavés. Les cellules sont alors traitées dans une solution sodée de façon à extraire la mélanine. Les solutions colorées sont alors dosées au spectrophotomètre à 450 nm pour leur teneur en mélanine grâce à une courbe d'étalonnage. Une activité inhibitrice de la mélanogénèse des produits testés se traduira par une teneur plus faible de mélanine dans les solutions extraites des mélanocytes. Un test préalable de cytotoxicité de ces mêmes cellules est bien sûr réalisé de façon à vérifier la non-toxicité des extraits aux concentrations testées.

L'activité d'inhibition de synthèse de la mélanine des différents produits testés est indiquée dans le tableau 1 ci-dessous.

**Tableau 1 : évaluation de l'activité sur la mélanogénèse d'un extrait de myrte selon l'invention**

| **Produits** | **Concentrations testées** | **% d'inhibition** |
|---|---|---|
| **Témoin** | - | - |
| Hydroquinone | 0,005 mM | 35,7 |
| | 0,01 mM | 58 |
| Extrait de myrte | 5 µg/ml | 41,7 |
| | 10 µg/ml | 43,7 |
| | 25 µg/ml | 39,4 |

L'hydroquinone montre bien une activité inhibitrice de la teneur en mélanine et ainsi de la mélanogénèse aux concentrations testées, et ainsi valide le test d'évaluation d'activité.

L'extrait de myrte présente une activité inhibitrice de la mélanogénèse aux 3 concentrations testées, et ainsi montre bien son intérêt dans la dépigmentation.

### Exemple 6 : Sérum cosmétique

| Composé | Quantité (/ 100g) |
|---|---|
| Extrait sec *Myrtus communis* (exemple 2) | 0,1 g à 0,5 g |
| Ascorbyl glucoside | 1,0 g à 10,0 g |
| Cyclométhicone | 2,0 g à 10,0 g |
| Carbomere | 0,5 g à 0,6 g |
| Propylène glycol | 1,0 g à 5,0 g |
| Glycérine | 1,0 g à 5,0 g |
| Triéthanolamine | 0,5 g à 2,5 g |
| Eau purifiée | Qsp 100 g |

### Exemple 7 : Etude de l'efficacité du sérum selon l'exemple 6

### • PREMIERE ETUDE

L'étude clinique présentée ci-après a été réalisée en ouvert, auprès de 30 femmes japonaises, âgées de 25 à 45 ans :
- ayant toutes la peau sensible (100%)
- et présentant des taches de pigmentation sur le visage, dont au moins une des taches avait un diamètre supérieur à 5 mm.

Parmi cet effectif, différentes natures de peau étaient représentées :
- peau normale (17 %)
- mixte grasse (26%)
- grasse (23%)
- mixte sèche (17%)
- sèche (17%).

Cette étude concerne la composition du sérum donnée précédemment en exemple 6. On note J0 le point de départ de l'étude (avant la première application du sérum). Le produit a été appliqué deux fois par jour le matin et le soir pendant 12 semaines (de J1 à J84) sur l'ensemble du visage et du cou, en insistant sur les taches pigmentaires.

Cette composition cosmétique est appliquée à la place du produit hydratant habituel.

Différentes techniques d'évaluation ont été utilisées :

### 1. Colorimétrie / paramètre de mesure de la pigmentation de la peau : index mélanique

L'index mélanique de la peau a été mesuré à l'aide du MEXAMETER® MX16 (Courage and Khazaka).

La sonde du mexamètre émet un rayonnement dans 3 longueurs d'onde sur la zone à mesurer, lequel est absorbé par la peau. Le diamètre de la surface de mesure est de 5 mm. Ensuite, un récepteur mesure la lumière réfléchie émise par la peau. Deux longueurs d'onde sont sélectionnées pour la mesure de la mélanine.

L'appareil indique alors un index mélanique, en unités arbitraires (u.a.), en fonction de la quantité de mélanine dans la peau à l'endroit de la mesure.

### PROTOCOLE

Zones de mesure : l'index mélanique de la peau a été mesuré au niveau d'une tache dont le diamètre est supérieur à 5mm, ainsi qu'au niveau d'une zone saine, à proximité de ladite tache.

Temps de mesure : les valeurs ont été relevées à J0 (avant la première application du sérum), ainsi qu'à J42 (après 42 jours de traitement) et J84 (après 84 jours de traitement).

### RESULTATS

Les résultats des mesures effectuées à chaque temps de mesure et sur chaque sujet ont fait l'objet d'une moyenne par zone (zone hyperpigmentée, zone peau saine).

On note Δ (exprimé en unités arbitraires (u.a.)) la différence entre l'index mélanique de la zone hyperpigmentée et l'index mélanique de la zone saine.
Le tableau 2 ci-dessous présente les résultats obtenus :

**Tableau 2: index mélaniques de la peau traitée avec le sérum à base d'un extrait de myrte**

| Temps des mesures | Δ index mélanique (en u.a.) (moyenne des mesures) |
|---|---|
| **J0** | 25,5 ± 2,9 |
| **J42** | 23,6 ± 2,9 |
| *J42 - J0 (en %)* | *-7,5*% |
| **J84** | 23,0 ± 3,1 |
| *J84 - J0 (en %)* | *-9%* |

Sur les 12 semaines de traitement, on observe que la valeur du Δ a significativement diminué par rapport à sa valeur initiale à J0 : diminution de 9 %; et ceci dès 6 semaines de traitement (J42) où une diminution du Δ de 7,5 % a été constatée.

Cela traduit raisonnablement une tendance de la composition testée à réduire l'index mélanique des taches pigmentaires.

La Demanderesse a ainsi mis en évidence que l'application d'une composition selon la présente invention induit une homogénéisation significative du teint.

### 2. Evaluation clinique

Un Dermatologue a constaté l'état initial de la peau de chaque sujet lors de l'entrée dans l'étude (J0) ; puis a suivi l'évolution de l'état cutané après 28 jours et après 84 jours de traitement.

Les paramètres étudiés sont les suivants:
- apparence des taches pigmentaires,
- éclat du teint,
- clarté du teint, et
- uniformité du teint.

### PROTOCOLE

Les différents paramètres ont été étudiés selon un « Clinical storing system » en 9 points :

| LEGER | | MODERE | | IMPORTANT | |
|---|---|---|---|---|---|
| + | 1 | + | 4 | + | 7 |
| ++ | 2 | ++ | 5 | ++ | 8 |
| +++ | 3 | +++ | 6 | +++ | 9 |

### RESULTATS

On note Δ₂₈ et Δ₈₄ les variations (exprimées en %) de chaque critère, respectivement à J28 et à J24 par rapport à leur valeur à J0.

A J0, J28 et J84, l'évaluation des critères est la suivante (tableau 3):

**Tableau 3 : étude clinique de 4 paramètres menée par un Dermatologue sur 30 femmes**

| *Paramètre étudié :* | valeur à J0 | valeur à J28 | Δ₂₈ | valeur à J84 | Δ₈₄ |
|---|---|---|---|---|---|
| apparence des taches | 5,2 ± 1,5 | 4,8 ± 1,6 | **- 8%** | 3,9 ± 1,4 | **-25%** |
| éclat du teint | 5,6 ± 1,9 | 6,7 ± 1,5 | **+ 20%** | 7,3 ± 1,3 | **+30%** |
| clarté du teint | 5,6 ± 1,8 | 6,3 ± 1,4 | **+ 13%** | 6,9 ± 1,6 | +**23**% |
| uniformité du teint | 5,8 ± 1,8 | 6,4 ± 1,4 | + **10%** | 6,9 ± 1,6 | +**19**% |

Au bout de 4 semaines (J28), et avantageusement au bout de 12 semaines (J84), on observe une variation favorable et significative de tous les critères évalués : c'est à dire des taches moins apparentes et un teint plus éclatant, plus clair et plus uniforme.

### CONCLUSION DE LA PREMIERE ETUDE D'EFFICACITE

Cette étude a permis de démontrer l'efficacité significative d'une composition comprenant un extrait de myrte selon la présente invention pour des peaux sensibles de différentes natures présentant des taches pigmentaires. Les bénéfices obtenus se traduisent par des taches moins apparentes et une amélioration du teint en terme de clarté, d'éclat et d'uniformité, d'homogénéisation.

### • DEUXIEME ETUDE

Cette deuxième étude clinique présentée ci-après a été réalisée en aveugle, auprès de 40 sujets japonais (37 femmes et 3 hommes), âgés de 22 à 48 ans.

Cette étude concerne la composition du sérum donnée précédemment en exemple 6.

On note J0 le point de départ de l'étude (avant la première application du sérum). Le produit a été appliqué une fois par jour, le soir après la toilette, pendant 3 mois (de J1 à J90) sur la face interne de l'avant bras (choisi selon randomisation).

### Technique d'évaluation utilisée : Colorimétrie / paramètre de mesure de la pigmentation de la peau : évolution du paramètre b*

Le paramètre b* (axe bleu-jaune) de la colorimétrie a été mesuré à l'aide du chromamètre CR 400® (Minolta).

La sonde du chromamètre émet un flash de lumière blanche sur la zone à mesurer, lequel est absorbé par la peau. Le diamètre de la surface de mesure est de 8 mm. Ensuite, 3 capteurs correspondant aux cônes de l'oeil humain enregistrent la lumière réfléchie par la peau. L'appareil indique alors les valeurs L*, a* et b*, en unités arbitraires (u.a.). Ces valeurs sont fonction de la quantité de mélanine dans la peau à l'endroit de la mesure.

### PROTOCOLE

Zones de mesure : la pigmentation cutanée a été mesurée au niveau de la face interne de l'avant bras (zone traitée), ainsi qu'au niveau d'une zone non traitée adjacente (zone témoin) - ces zones de mesures étant préalablement délimitées et repérées à l'aide de masques de repérages.

Temps de mesure : les valeurs ont été relevées à J0 (avant la première application du sérum), à J30 (après 1 mois de traitement), J60 (après 2 mois de traitement) et J90 (après 3 mois de traitement).

### RESULTATS

Les résultats des mesures effectuées à chaque temps de mesure et sur chaque sujet ont fait l'objet d'une moyenne par zone (zone traitée, zone témoin).

Afin d'évaluer les modifications de coloration cutanée suite à l'application du sérum selon la présente invention, on exprime pour chaque temps de mesure (J30, J60, J90) la valeur de b* pour chaque zone, ainsi que la différence entre les valeurs colorimétriques de la zone traitée par rapport à la zone témoin.

**Tableau 4: paramètre b* colorimétrique de la peau traitée avec le sérum à base d'un extrait de myrte et de la zone témoin adjacente**

| Temps de mesure | b* moyenne (en u. a.) zone **traitée** | b* moyenne (en u. a.) zone **témoin** | b*_{zone traitée} -b*_{zone témoin} | % évolution des moyennes (b*_{zone traitée} - b* _{zone témoin}) par rapport à J0 |
|---|---|---|---|---|
| **J0** | 18,52 | 17,18 | 1,34 | - |
| **J30** | 18,20 | 16,97 | 1,23 | **-18,7%** |
| **J60** | 17,25 | 15,78 | 1,47 | **+ 4,5 %** |
| **J90** | 16,96 | 16,28 | 0,68 | **- 44,6 %** |

D'une part, on observe que la valeur b* de la zone traitée diminue progressivement et nettement tout au long de l'étude jusqu'à J90, ce qui traduit une décoloration de la zone traitée.

D'autre part, après 3 mois de traitement (J90), on observe une diminution significative par rapport à J0 de la différence (b* _{zone traitée} - b* _{zone témoin}) ; soit une diminution de 44,6 % de cette différence. Cela démontre raisonnablement une tendance de la composition testée à réduire la pigmentation cutanée.

### CONCLUSION DE LA DEUXIEME ETUDE D'EFFICACITE

La Demanderesse a ainsi mis en évidence que l'application d'une composition selon la présente invention a un effet dépigmentant.

## Revendications

1. Utilisation cosmétique d'un extrait de myrte (*Myrtus* communis) comme agent dépigmentant dans une composition cosmétique.

2. Utilisation selon la revendication 1, **caractérisée en ce que** la composition est destinée à blanchir et/ou éclaircir la peau et/ou les poils et/ou les cheveux.

3. Utilisation selon la revendication 1, **caractérisée en ce que** la composition est destinée à réduire et/ou supprimer les taches de sénescence de la peau.

4. Utilisation selon la revendication 1, **caractérisée en ce que** la composition est destinée à réduire et/ou supprimer les taches pigmentaires brunâtres pouvant être induites par les UV ou les chloasmas.

5. Utilisation selon l'une quelconque des revendications 1 à 4, **caractérisée en ce que** la quantité d'extrait de myrte est comprise entre 1 mg et 50 g pour 100 g de composition.

6. Utilisation selon l'une quelconque des revendications 1 à 5, **caractérisée en ce que** la composition comprend au moins un autre agent dépigmentant dans un milieu physiologiquement acceptable.

7. Utilisation selon la revendication 6, **caractérisée en ce que** l'autre agent dépigmentant est choisi dans le groupe constitué par la vitamine C et ses dérivés, tels que l'ascorbyl glucoside, l'acide salicylique, l'acide lactique, l'acide glycolique, l'acide malique, l'arbutine, l'acide kojique, la vitamine B3, l'acide linoléique et les huiles végétales en contenant, le rétinaldéhyde et ses dérivés, le résorcinol, un extrait de busserole, un extrait de réglisse, un extrait de mûrier, un extrait d'arbousier, un extrait de broussonetia, un extrait de camomille et un extrait de scutellaire, utilisé seul ou en mélange.

## Patentansprüche

1. Kosmetische Verwendung eines Myrtenextrakts (*Myrtus* communis) als depigmentierendes Mittel in einer kosmetischen Zusammensetzung.

2. Verwendung nach Anspruch 1, **dadurch gekennzeichnet, dass** die Zusammensetzung zum Bleichen und/oder Aufhellen der Haut und/oder der Behaarung und/oder des Haars bestimmt ist.

3. Verwendung nach Anspruch 1, **dadurch gekennzeichnet, dass** die Zusammensetzung zum Reduzieren und/oder Entfernen der Altersflecke der Haut bestimmt ist.

4. Verwendung nach Anspruch 1, **dadurch gekennzeichnet, dass** die Zusammensetzung zum Reduzieren und/oder Entfernen der bräunlichen Pigmentflecke, die von den UV oder den Chloasmen hervorgerufen sein können, bestimmt ist.

5. Verwendung nach einem der Ansprüche 1 bis 4, **dadurch gekennzeichnet, dass** die Myrtenextraktmenge zwischen 1 mg und 50 g inklusive auf 100 g Zusammensetzung beträgt.

6. Verwendung nach einem der Ansprüche 1 bis 5, **dadurch gekennzeichnet, dass** die Zusammensetzung mindestens ein anderes depigmentierendes Mittel in einem physiologisch akzeptablen Milieu umfasst.

7. Verwendung nach Anspruch 6, **dadurch gekennzeichnet, dass** das andere depigmentierende Mittel aus der Gruppe ausgewählt ist, die von dem Vitamin C und seinen Derivaten wie dem Ascorbylglucosid, der Salicylsäure, der Milchsäure, der Glycolsäure, der Maleinsäure, dem Arbutin, der Kojisäure, dem Vitamin B3, der Linolsäure und den Pflanzenölen, die davon enthalten, dem Retinaldehyd und seinen Derivaten, dem Resorcinol, einem Bärentraubeextrakt, einem Süßholzextrakt, einem Maulbeerextrakt, einem Erdbeerbaumextrakt, einem Broussonetiaextrakt, einem Kamilleextrakt und einem Helmkrautextrat, verwendet allein oder im Gemisch, gebildet ist.

## Claims

1. Cosmetic use of a myrtle extract (*Myrtus communis*) as depigmenting agent in a cosmetic composition.

2. Use according to claim 1, **characterized in that** the composition is intended to whiten and/or lighten the skin and/or body hair and/or head hair.

3. Use according to claim 1, **characterized in that** the composition is intended to reduce and/or eliminate skin age spots.

4. Use according to claim 1, **characterized in that** the composition is intended to reduce and/or eliminate brownish pigment marks which may be induced by UVs, or chloasmas.

5. Use according to any of claims 1 to 4, **characterized in that** the quantity of myrtle extract lies between 1 mg and 50 g per 100 g of composition.

6. Use according to any of claims 1 to 5, **characterized in that** the composition comprises at least one other depigmenting agent in a physiologically acceptable medium.

7. Use according to claim 6, **characterized in that** the other depigmenting agent is chosen from the group consisting of vitamin C and its derivatives such as ascorbyl glucoside, salicylic acid, lactic acid, glycolic acid, malic acid, arbutin, kojic acid, vitamin B3, linoleic acid and vegetable oils containing the same, retinaldehyde and its derivatives, resorcinol, bearberry extract, liquorice extract, mulberry extract, arbutus extract, Broussonetia extract, camomile extract and Scutellaria extract, used alone or in a mixture.
